(19) Europäisches Patentamt
European Patent Office
Office européen des brevets

(11) EP 2 113 518 A1

(12) **EUROPEAN PATENT APPLICATION**
published in accordance with Art. 153(4) EPC

(43) Date of publication:
**04.11.2009 Bulletin 2009/45**

(21) Application number: **08710469.1**

(22) Date of filing: **22.02.2008**

(51) Int Cl.:
***C08B 37/00*** (2006.01) ***A61K 31/726*** (2006.01)
***A61P 19/10*** (2006.01)

(86) International application number:
**PCT/JP2008/000316**

(87) International publication number:
**WO 2008/102568 (28.08.2008 Gazette 2008/35)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **22.02.2007 JP 2007042272**

(71) Applicants:
- **PG Research Co., Ltd.**
  **Tokyo 187-0002 (JP)**
- **Tohoku University**
  **Aoba-ku**
  **Sendai-shi**
  **Miyagi 980-8577 (JP)**

(72) Inventors:
- **MIYAZAKI, Tatsuya**
  **Kodaira-shi**
  **Tokyo 187-0002 (JP)**
- **MIYAUCHI, Satoshi**
  **Kodaira-shi**
  **Tokyo 187-0002 (JP)**
- **MATSUZAKA, Satoshi**
  **Kodaira-shi**
  **Tokyo 187-0002 (JP)**
- **SUZUKI, Osamu**
  **Sendai-shi**
  **Miyagi 980-0812 (JP)**

(74) Representative: **Gille Hrabal Struck Neidlein Prop Roos**
**Brucknerstraße 20**
**40593 Düsseldorf (DE)**

# (54) BONE/CARTILAGE FORMATION-STIMULATING AGENT

(57) Disclosed is a mixture of: a bone/cartilage formation promoting agent containing sulfated galactosaminoglycan having greater than or equal to 0.6 numbers of ester sulfate groups on average per constituent monosaccharide or a salt thereof as an active ingredient; a factor having a bone/cartilage formation promoting action; or a bone filler (BMP, TGF-β, FGF, IGF, insulin, PDGF, HGF, midkine, pleiotrophin, collagen, gelatin, proteoglycan, fibronectin, osteocalcin, osteopontin, osteonectin, bone sialoprotein, hydroxyapatite, dicalcium phosphate anhydride, dicalcium phosphate dehydrate, α-tricalcium phosphate, amorphous calcium phosphate, octacalcium phosphate, β-tricalcium phosphate, PLLA, PLGA, titanium, decalcified bone, autogeneous bone, or the like).

[FIG. 1]

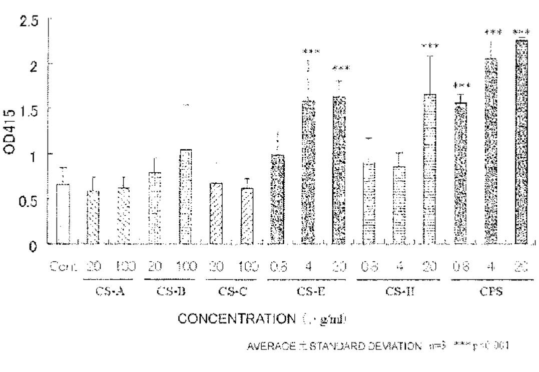




Printed by Jouve, 75001 PARIS (FR)

## Description

Technical Field

The present invention relates to a bone/cartilage formation promoting agent which can be used when a bone tissue and a cartilage tissue are damaged.

Background Art

Regarding treatments of bone fracture, a method of resetting and fixing a damaged part and causing it to be naturally cured is general. At this time, pharmacologic treatments are hardly carried out except the cases where patients have brittle-bone disease or diabetes. Regarding a wide-range damage of a bone/cartilage tissue and a partial deficiency (after a bone tumor removing operation, and, lip and palate), an artificial bone filler is filled or a bone/cartilage tissue is implanted.

It is reported that bone morphogenetic proteins (BMPs), a transforming growth factor-β (TGF-β), a fibroblast growth factor (FGF), an insulin-like growth factor (IGF), a Midkine (MK) and the like are substances which promote cell growth or cell differentiation of a damaged or deficient bone/cartilage.

Moreover, matrix components which promote bone/cartilage formation, such as collagens, gelatins, hydroxyapatite of bone fillers, calcium phosphate like β-tricalcium phosphate or octacalcium phosphate (see patent literatures 1 and 2), decalcified bones, and autogenic bones, are used.

Furthermore, it is known that a mixture of the foregoing growth factors and the bone fillers is effective for restoring a deficient part.

Regarding glucosaminoglycan, it is reported that a chondroitin sulfate (see non-patent literature 1) and heparin (see non-patent literature 2) promote differentiation of osteoblastic cells, and heparin and a heparan sulfate are effective for bone formation as an action augmenting agent of BMPs (see non-patent literature 3). Moreover, a mixture (see patent literature 3) of hydroxyapatite and chondroitin sulfate or a mixture (see patent literature 4) of β-tricalcium phosphate and a chondroitin sulfate are also reported.

Patent Literature 1: JPH05-70113A
Patent Literature 2: JP2006-167445A
Patent Literature 3: JPH08-229114A
Patent Literature 4: JPH08-229113A
Non-patent Literature 1: Bouvier M., et al, Arch Oral Biol 35(4), 301-309. 1990
Non-patent Literature 2: Hausser H-J, et al, J Cell Biochem 91, 1062-1073, 2004
Non-patent Literature 3: Zhao B., et al, J Biol Chem 281(32): 23246-53, 2006

Disclosure of Invention

Problem to be Solved by the Invention

With respect to a bone fracture and a wide-range damage of a bone/cartilage tissue, even if bone filling or bone/cartilage implantation is carried out, fixing for a long term is needed conventionally after such a treatment is carried out. Such long-term fixing causes a serious problem such that a patient, in particular, an elderly adult, becomes bedridden sometimes. Accordingly, there is a demand to accomplish regeneration and curing of a bone/cartilage as quick as possible.

Means for Solving the Problem

The inventors of the present invention keenly studied to overcome the foregoing problem, and as a result, found out that galactosaminoglycan with a specific structure promotes growth and differentiation of a cultured osteoblastic cell and collagen synthesis of a cultured cartilage cell, thus having a superior bone/cartilage formation promoting action, and accomplished the present invention.

That is, the scope of the present invention is as follows.

(1) A bone/cartilage formation promoting agent comprising, as an active ingredient, sulfated galactosaminoglycan or a pharmacologically acceptable salt thereof, the sulfated galactosaminoglycan containing greater than or equal to 0.6 numbers of ester sulfate groups on average per constituent monosaccharide.

(2) The bone/cartilage formation promoting agent described in (1), wherein sulfated galactosaminoglycan is sulfated galactosaminoglycan containing an N-acetylchondrosine or N-acetyldermosine structure.

(3) The bone/cartilage formation promoting agent described in (1) or (2), wherein sulfated galactosaminoglycan has a structure of any one of formulas 1 to 4.

[Chemical Formula 1]

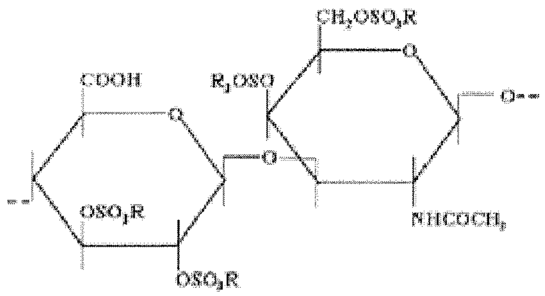

( Formula 1 )

[Chemical Formula 2]

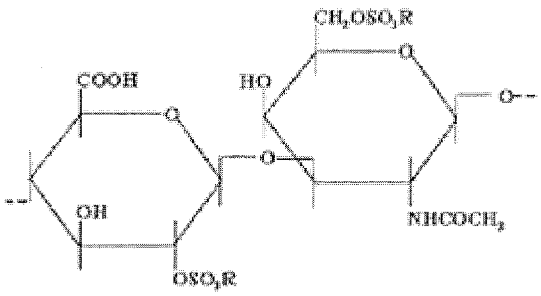

( Formula 2 )

[Chemical Formula 3]

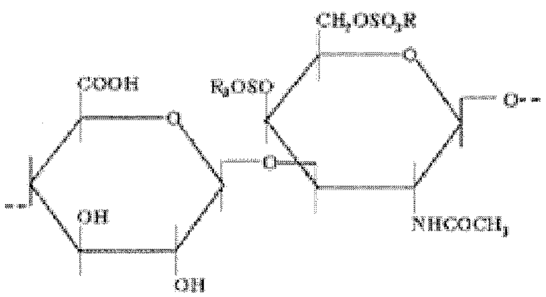

( Formula 3 )

[Chemical Formula 4]

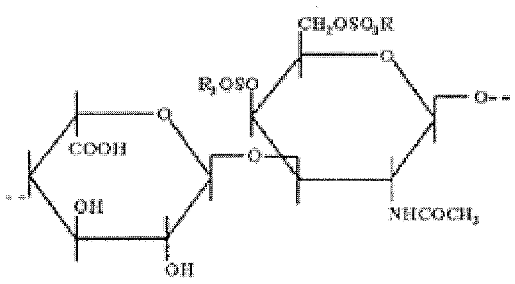

( Formula 4 )

(where C, O, H, N and S in formulas represent a carbon atom, an oxygen atom, a hydrogen atom, a nitrogen atom, and a sulfur atom, respectively, and R represents a hydrogen atom or a substituent thereof.)

(4) The bone/cartilage formation promoting agent described in any one of (1) to (3), wherein a molecular weight of sulfated galactosaminoglycan is greater than or equal to 1000 Da.

(5) The bone/cartilage formation promoting agent described in any one of (1) to (4), wherein a content rate of the structure of any one of formulas 1 to 4 is greater than or equal to 20 mol%.

[Chemical Formula 5]

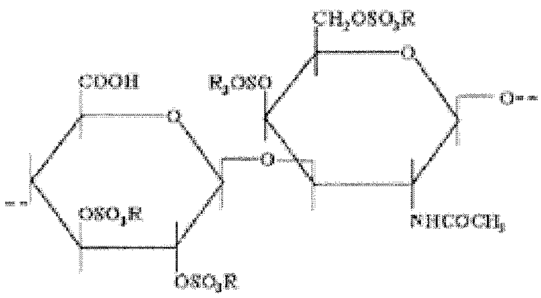

( Formula 1 )

[Chemical Formula 6]

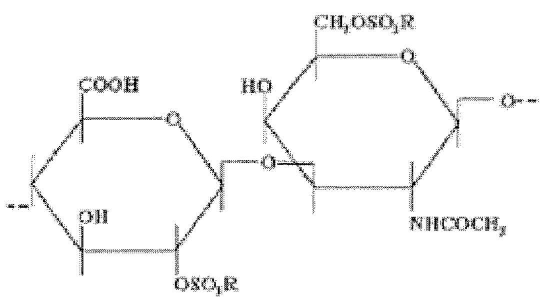

( Formula 2 )

[Chemical Formula 7]

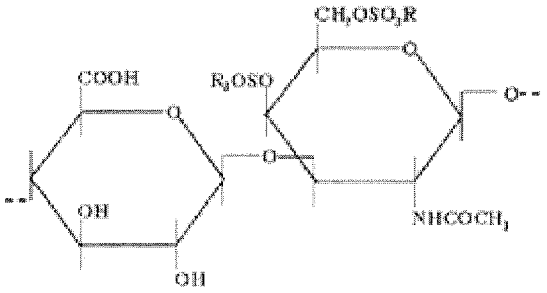

( Formula 3 )

[Chemical Formula 8]

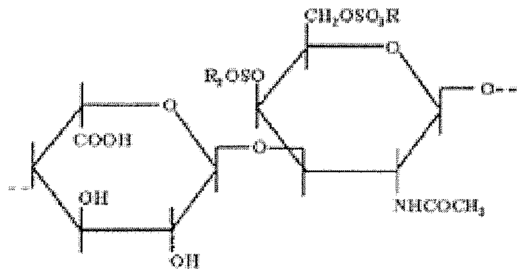

( Formula 4 )

(where C, O, H, N and S in formulas represent a carbon atom, an oxygen atom, a hydrogen atom, a nitrogen atom, and a sulfur atom, respectively, and R represents a hydrogen atom or a substituent thereof.)

(6) A bone/cartilage formation promoting agent comprising, as an active ingredient, sulfated galactosaminoglycan originating from a cartilage of cephalopoda or selachii, or a chorda dorsalis of agnathonae.

(7) The bone/cartilage formation promoting agent described in any one of (1) to (6), further comprising, as an additive, a factor of promoting bone formation or a bone filler.

(8) The bone/cartilage formation promoting agent described in (7), wherein the factor of promoting bone formation or the bone filler is at least one substance selected from BMP, TGF-β, FGF, IGF, insulin, PDGF, HGF, midkine, collagen, gelatin, proteoglycan, fibronectin, osteocalcin, osteopontin, osteonectin, bone sialoprotein, matrix Gla protein, hydroxyapatite, dicalcium phosphate anhydride, dicalcium phosphate dehydrate, α-tricalcium phosphate, amorphous calcium phosphate, octacalcium phosphate, β-tricalcium phosphate, poly-L-lactic acid (PLLA), lactic-acid-glycolic-acid copolymer (PLGA), titanium, decalcified bone, and autogeneous bone.

(9) A pharmaceutical composition comprising the bone/cartilage formation promoting agent of any one of (1) to (8) for cartilage formation failure and cartilage formation.

(10) A method of using sulfated galactosaminoglycan or a pharmacologically acceptable salt thereof for promoting bone/cartilage formation, the sulfated galactosaminoglycan containing greater than or equal to 0.6 numbers of ester sulfate groups on average per constituent monosaccharide.

(11) A method of using sulfated galactosaminoglycan or a pharmacologically acceptable salt thereof for promoting cartilage formation, the sulfated galactosaminoglycan containing greater than or equal to 0.6 numbers of ester sulfate groups on average per constituent monosaccharide.

Effect of the Invention

According to the present invention, there is provided a new bone/cartilage formation promoting agent which contains sulfated galactosaminoglycan as an active ingredient.

Brief Description of Drawings

FIG. 1 is a diagram showing a result of calcification promoting action of a cultured osteoblastic cell MC3T3-E1 by polysulfated galactosaminoglycan;
FIG. 2 is a diagram showing a result of calcification promoting action of a cultured osteoblastic cell MC3T3-E1 by polysulfated galactosaminoglycan;
FIG. 3 is a diagram showing a result of alkaline phosphatase activity prokinetic action of a cultured osteoblastic cell MC3T3-E1 by polysulfated galactosaminoglycan;
FIG. 4 is a diagram showing a result of growth promoting action of a cultured osteoblastic cell MC3T3-E1 by polysulfated galactosaminoglycan;
FIG. 5 is a diagram showing a result of calcification promoting action of an osteoblastic cell MC3T3-E1 by polysulfated galactosaminoglycan, wherein (A) shows a culturing over octacalcium phosphate (OCP) and (B) shows a culturing over hydroxyapatite (HA);
FIG. 6 is a diagram showing a result of growth promoting action of an osteoblastic cell MC3T3-E1 by polysulfated galactosaminoglycan, wherein (A) shows a culturing over octacalcium phosphate (OCP) and (B) shows a culturing over hydroxyapatite (HA);
FIG. 7 is a diagram showing a result of collagen synthesis promoting action of a cultured swine cartilage cell by CS-E. FIG. 7 also shows a collagen content per twenty alginate gelbeades; and
FIG. 8 shows a result of collagen synthesis promoting action of a cultured swine cartilage cell by CS-E. FIG. 8 also shows a collagen content per DNA.

Best Mode for Carrying Out the Invention

The present invention will be explained in detail through the best mode for carrying out the present invention.

A medicine of the present invention is a bone/cartilage formation promoting agent containing, as an active ingredient, sulfated galactosaminoglycan which contains greater than or equal to 0.6 numbers of ester sulfate groups on average per constituent monosaccharide, or a salt thereof which is pharmaceutically acceptable.

Galactosaminoglycan in the medicine of the present invention is acidic polysaccharide containing galactosamine, and normally, has a basic skeleton which is an iteration structure of galactosamine and uronic acid. Uronic acid is generally glucuronic acid or iduronic acid. Most galactosaminoglycan present in nature are chondroitin sulfate A (hereinafter, CS-A) or chondroitin sulfate B (hereinafter, CS-B, another name is dermatan sulfate) having a sulfate group combined with the position 4 of N-acetylgalactosamine, and chondroitin sulfate C (hereinafter, CS-C) having a sulfate group combined with the position 6 of N-acetylgalactosamine. An active ingredient of the medicine of the present invention is sulfated galactosaminoglycan (hereinafter, polysulfated galactosaminoglycan) containing a structure having greater than or equal to two sulfate groups in a repeating disaccharide unit, such as synthesis polysulfated chondroitin sulfate (hereinafter, CPS, see formula 1) having a sulfate group combined with the position 2 and the position 3 of glucuronic acid and the position 4 and the position 6 of N-acetylgalactosamine in a disaccharide structure of glucuronic acid and N-acetylgalactosamine, chondroitin sulfate D (hereinafter, CS-D, see formula 2) having a sulfate group combined with the position 2 of glucuronic acid and the position 6 of N-acetylgalactosamine, chondroitin sulfate E (hereinafter, CS-E,

see formula 3) having a sulfate group combined with the position 4 and the position 6 of N-acetylgalactosamine, and chondroitin sulfate H (hereinafter, CS-H, see formula 4) having a sulfate group combined with the position 4 and the position 6 of N-acetylgalactosamine in a disaccharide structure of iduronic acid and N-acetylgalactosamine.

[Chemical Formula 9]

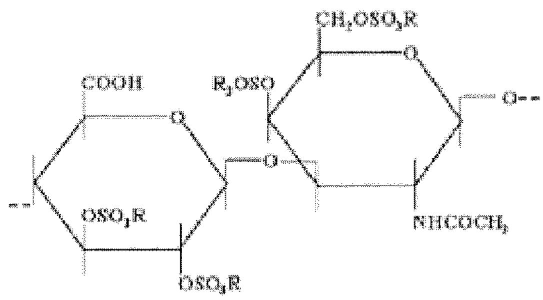

( Formula 1 )

[Chemical Formula 10]

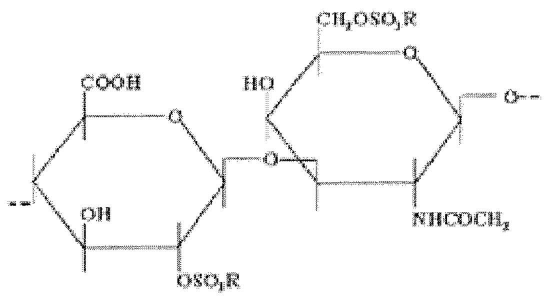

( Formula 2 )

[Chemical Formula 11]

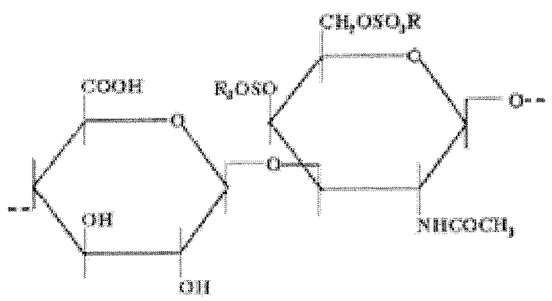

( Formula 3 )

[Chemical Formula 12]

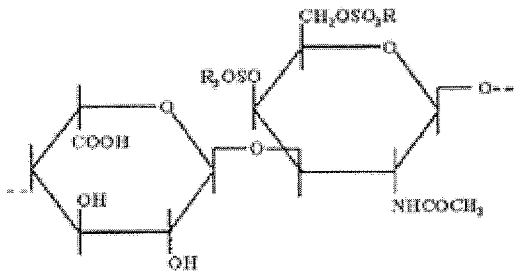

( Formula 4 )

Polysulfated galactosaminoglycan acquired from natural sources is not limited to any particular one if it can be acquired by extraction and purification from an organism (e.g., an animal tissue) containing galactosaminoglycan through an ordinary technique (a simple combination of a physical extraction technique, an enzyme extraction technique, an organic solvent fractionation technique, and a chromatography fractionation technique using an ion-exchange resin). When polysulfated galactosaminoglycan is extracted and purified from an organism, the kind of an animal and the kind of a tissue are not limited to any particular ones, but polysulfated galactosaminoglycan extracted and purified from, for example, tissues (cartilage, bone, chorda dorsalis, brain, bowel, medulla spinalis, and the like) of a shark, a squid, an octopus, a salmon, a borer, a cattle, and a swine can be used.

An example of an extraction technique from such tissues is a technique of performing homogenation on such a tissue by a salt solution or an acetic acid dilute, or digesting such a tissue by protease, performing centrifuge separation, and then acquiring rough polysulfated galactosaminoglycan in a supernatant. In order to remove contaminating proteins, it is desirable to digest a tissue using protease, such as papain, pronase, or actinase. The following is a general technique as a purification technique from a rough extraction liquid. Sodium acetate, calcium acetate, or sodium chloride is dissolved in the rough extraction liquid, ethanol which is three times as much as an equivalent amount of the extraction liquid is added while the extraction liquid is stirred to cause rough polysulfated galactosaminoglycan to be precipitated. A technique of adding quarternary ammonium salt, such as benzalkonium chloride (Osvan) or cetylpyridinium chloride (CPC), to the rough extraction liquid to cause polysulfated galactosaminoglycan to be precipitated can be used independently or together with precipitation by ethanol. After the precipitate is rinsed by ethanol, the precipitate is processed using an alkaline aqueous solution like a sodium hydroxide solution to remove peptide combined with a reducing end of sulfated galactosaminoglycan. The process using the alkaline aqueous solution is not needed when peptide combined with the reducing end is not a problem, but in order to avoid the immunogenic potential originating from peptide, it is desirable to carry out such a process. When the process using the alkaline aqueous solution is carried out, neutralization by a dilute hydrochloric acid solution or the like is carried out, the liquid is loaded to an anion exchanger column, and chromatography is carried out. The anion exchanger is not limited to any particular one, but examples of such exchanger are a dowex anion exchanger resin (the dow chemical company), an amberlite anion exchanger resin (Rohm and Haas company), an AG anion exchanger resin (Bio-rad company), diethylaminoethyl (DEAE) sepharose (GE healthcare bio science company). Polysulfated galactosaminoglycan is eluted through a gradient technique by an aqueous solution of sodium salt or a stepwise technique, and the eluted solution is fractionated by a fraction collector. Polysulfated galactosaminoglycan is detected through, for example, a carbazole sulfate technique (see Bitter T., et al, Anal Biochem 4, 330-334, 1962), and a target fraction is collected. Salts are removed by dialysis, gel filtration, or the like to acquire a sodium salt of purified polysulfated galactosaminoglycan. When elution is carried out by salts other than sodium, a corresponding salt of polysulfated galactosaminoglycan can be acquired. Anion exchanger chromatography is effective for purification, but if conditions of precipitation by quarternary ammonium salt and precipitation by ethanol are optimized, highly-purified polysulfated galactosaminoglycan can be acquired without carrying out chromatography.

It is desirable that CS-D among polysulfated galactosaminoglycans should be extracted from a shark cartilage. CS-E extracted from a cartilage tissue of a mollusk is preferable, and one extracted from a squid cartilage is more preferable. CS-H originating from a chorda dorsalis of a lower animal like a borer is preferable. Polysulfated galactosaminoglycan can be synthesized by chemical modification, such as enzymatically or chemically sulfating it from CS-A, CS-C. For example, CS-E can be synthesized by using chondroitin-6-sulfotransferase which is enzyme selectively transferring a sulfate group to the position 6 of an N-acetyl-galactosamine residue to CS-A.

The mass average molecular weight of polysulfated galactosaminoglycan is not limited to any particular one, but is preferable from 1000 Da to 150,000 Da, and is more preferable from 10,000 Da to 150,000 Da. However, it is generally known that the average molecular weight of glycosaminoglycan varies depending on a measuring technique, a measuring condition, and the like even if the same sample is used, so that the present invention should not be strictly

limited to the foregoing average molecular weight ranges.

A polysulfated structure of polysulfated galactosaminoglycan which is an active ingredient of the medicine of the present invention can be identified and quantified through disaccharide analysis. For example, a process is carried out with enzyme which acts on sulfated polysaccharide and forms unsaturated disaccharide, and unsaturated disaccharide which is formed by reflecting a structural disaccharide of the sulfated polysaccharide is analyzed through high performance liquid chromatography (HPLC). For example, 2-acetamide-2-deoxy-3-O-(2-O-sulfo-β-D-gluco-4-enopyranosyl uronic acid)-6-O-sulfo-D-galactose ($\Delta$Di-$S_D$) can be formed from CS-D, and 2-acetamide-2-deoxy-3-O-(β-D-gluco-4-enopyranosyl uronic acid)-4, 6-di-O-sulfo-D-galactose ($\Delta$Di-$S_E$) is formed from CS-E and CS-H, and those can be analyzed through HPLC (Yoshida K,. et al,: Anal Biochem 177, 327-332, 1989). Note that Δ means unsaturated disaccharide.

Enzyme used in disaccharide analysis is not limited to any particular one if it can be degraded to unsaturated disaccharide. It can be selected appropriately in accordance with the kind of sulfated polysaccharide to be analyzed. Examples of such enzyme are chondroitinase, hyaluronidase.

The foregoing technique through HPLC is carried out by comparing an elution position of unsaturated disaccharide acquired by performing enzyme process on sulfated polysaccharide with an elution position of standard unsaturated disaccharide. A kind of polysulfated structure and a content percentage thereof can be analyzed through the foregoing disaccharide analysis. It is preferable that polysulfated galactosaminoglycan which is an active ingredient of the medicine of the present invention should have a content percentage of a D structure, an E structure, or an H structure from 20 mol% to 100 mol%, more preferably, from 30 mol% to 100 mol%, and further preferably, from 50 mol% to 100 mol% through disaccharide analysis.

In a case of sodium salt, it is preferable that a sulfur content in polysulfated galactosaminoglycan should be greater than or equal to 7 %, and more preferably, should be greater than or equal to 8 %. When all sulfate groups and carboxyl groups in polysulfated galactosaminoglycan are present as sodium salts, 0.6 numbers of ester sulfate groups on average per component monosaccharide have 7.33 % sulfur content. In a case of sodium salt, a molecular weight of a basic structure ofN-acetyl chondrosine or N-acetyl dermosine is 401. A molecular weight of sodium salt of a sulfate group is 103, and an atomic weight of sulfur is 32. Since 0.6 numbers of ester sulfate groups on average per component monosaccharide are equivalent to 1.2 numbers of ester sulfate groups on average per component disaccharide, in a case of polysulfated galactosaminoglycan having 0.6 numbers of ester sulfate groups on average per component monosaccharide, a sulfur content can be acquired through the following equation.

Sulfur content (weight%) = 32 (atomic mass of sulfur) × 1.2 (number of ester sulfate groups per component disaccharide) / (401 (molecular weight of sodium salt of basic structure) + (103 (molecular weight of sodium salt of ester sulfate) × 1.2 (number of ester sulfate groups per component disaccharide) − 1 (atomic weight of hydrogen lost at the time of ester combination) ) × 100.

Polysulfated galactosaminoglycan used for the medicine of the present invention is not limited to one which is straight chained, but may be branching one.

Examples of salts pharmaceutically acceptable to polysulfated galactosaminoglycan are sodium salts, potassic salts, calcium salts, barium salts, metallic salts like magnesium salts or aluminum salts, amino acid salts, and amino sugar salts, and in particular, sodium salts are preferable. What is important in the present invention is that the medicine of the present invention contains polysulfated galactosaminoglycan as an active ingredient, and such medicine may be a mixture of different kinds of polysulfated galactosaminoglycans.

The medicine of the present invention has a remarkable tissue-recovery promoting action to a damage or partial deficiency of a bone/cartilage tissue of mammals, such as human, dogs, cats, horses, and rabbits, and can be applied to a treatment of bone illnesses, such as a bone fracture, and a bone deficiency (after bone tumor removal operation, and lip and palate) and illnesses, such as osteoarthritis, traumatic chondropathy, arthrorheumatism, meniscus injury, shoulder periarthritis, and jaw arthritis, but in particular, it is expected that the medicine of the present invention has a superior effect to illnesses which require promotion of formation of a cartilage and regeneration thereof.

Regarding how to administer the medicine of the present invention in vivo, it is not limited to any particular technique if the bone/cartilage formation promoting action of the medicine of the present invention is not deteriorated.

It can be appropriately selected depending on a characteristic of a target patient and a degree of seriousness thereof, and it is desirable that the medicine of the present invention should be in the dosage form which can be administered to a damaged part.

For example, with respect to a bone fracture and a bone deficiency, it is preferable that the medicine of the present invention should be directly given and attached to an affected part at the time of a surgery. By administering the medicine as a mixture with a factor which promotes bone formation in vivo or with a bone filler, it is expected that the foregoing effects can be further accelerated. The medicine of the present invention may be given by directly injecting it in the vicinity of an affected part or by subcutaneous infusion.

Examples of such factors which promote bone formation or bone fillers are BMP (Wozney JM., et al, Prog Growth Factor Res 1(4), 267-280, 1989, etc.,), TGF-β (Joyce ME., et al, J Cell Biol 110(6), 2195-2207, 1990, etc.,), FGF (Mayahara H., et al, Growth Factors 9(1), 73-80, 1993, etc.,), IGF (Mueller K., et al, Am J Physiol 267(1 Pt 1), E1-6, 1994, etc.,), insulin (Cornish J., et al, Calcif Tissue Int 59(6), 492-495, 1996, etc.,), PDGF (Vikjaer D., et al, Eur J Oral Sci 105(1), 59-66, 1997, etc.,), HGF (Amano O., Arch Oral Biol 44(11), 935-946, 1999, etc.,), midkine (Ohta S., et al, J Bone Miner Res 14(7) 1132-1144, 1999, etc.,), pleiotrophin (Imai S., et al, J Cell Biol 143(4), 1113-1128, 1998, etc.,), collagen (Saadeh PB., et al, J Craniofac Surg 12(6), 573-579, 2001, etc.,), gelatin, proteoglycan (Gomes RR. Jr, et al, Connect Tissue Res 44(1), 196-201, 2003, etc.,), fibronectin, osteocalcin, osteopontin, osteonectin, bone sialoprotein, hydroxyapatite, diculcium phosphate anhydride, dicalcium phosphate dihydrate, α-triculcium phosphate, amorphous calcium phosphate, octacalcium phosphate, β-tricalcium phosphate, poly-L-lactic acid (PLLA), lactic-acid-glycolic-acid copolymer (PLGA), titanium, decalcified bone, and autogenous bone. The medicine of the present invention may be a combination of any one or greater than or equal to two such factors.

When a damaged or deficient part is large, it is desirable to use, as a carrier of a bone filler or the medicine of the present invention, hydroxyapatite having a high biocompatibility, autogenous bone, calcium phosphate having functions of intravital absorption and bone substitution, such as β-tricalcium phosphate or octacalcium phosphate, and decalcified bone. By mixing those with the medicine of the present invention, a bone/cartilage formation effect of polysulfated galactosaminoglycan can be accelerated.

Regarding how to produce such a mixture, a technique of mixing a matrix component and polysulfated galactosaminoglycan in an aqueous solution or in a gel is simple and desirable, but a technique of chemically combining those can be used if it does not lose the activities of both matrix component and polysulfated galactosaminoglycan. Moreover, a solid material can become available by freezing and drying the mixture liquid of both matrix component and polysulfated galactosaminoglycan. When producing a sponge-like mixture by freezing and drying, in order to delay dissipation at an administered portion, it is desirable to cause the matrix component to be insoluble by cross-linking or the like. In a case of collagen, a sponge-like carrier can be produced by performing heating, ultraviolet exposure or chemical cross-liking on atelocollagen having a low antigenecity.

Regarding how to mix a bone filler and polysulfated galactosaminoglycan, it is possible to soak a bone filler in a polysulfated galactosaminoglycan solution to combine both components. In a case of hydroxyapatite or calcium phosphate, a sulfate group or a carboxyl group of polysulfated galactosaminoglycan can be fixed to such calcium by ionic bonding. Furthermore, by freezing and drying a mixture of a bone filler and polysulfated galactosaminoglycan, a further larger amount of polysulfated galactosaminoglycan can be fixed. A bone filler to be used may be in any well-known forms, such as a granular body, a porous body, and a dense body, and can be selected depending on a use purpose and a portion where the medicine of the present invention is used. An impregnating agent of a bone filler in the form of a granular body with polysulfated galactosaminoglycan can also be used.

First Example

For an osteoblastic cell test, the following galactosaminoglycan were purchased and used.
Chondroitin sulfate A (CS-A, originating from a whale cartilage, a sulfur content was 6.32 %, and made by SEIKAGAKU corporation), chondroitin sulfate B (CS-B, originating from a hog skin, a sulfur content was 6.57 % and made by SEIKAGAKU corporation), chondroitin sulfate C (CS-C, a chondron injection solution, made by KAKEN pharmaceutical corporation), chondroitin sulfate E (CS-E, originating from a squid cartilage, E structure was 64.9 %, a sulfur content was 8.75 %, made by SEIKAGAKU corporation), chondroitin sulfate H (CS-H, originating from borer chorda dorsalis, made by PG research corporation) and polysulfated chondroitin sulfate (CPS, adequan, made by SANKYO lifetech corporation) were dissolved and diluted by a phosphate buffered saline (PBS (-)), and added to a culture medium.

Using an osteoblastic cell strain MC3T3-E1 originating from a mouse skull bone, an action to osteoblactic cell differentiation was checked. MC3T3-E1 cell was cultured on α-MEM (growth medium) containing 5 % fetal bovine serum (FBS) and 10 μg/ml gentamicin. When the cell became confluent, the cell was collected using 0.25 % trypsin solution, and was subjected to seeding to a 48-well plate at $1 \times 10^4$/0.5 mL/well with the growth medium. After 24 hours passed from the cultivation, the culture medium was replaced with a growth medium (differentiation medium) containing 50 μg/mL ascorbic acid, 10 mmol/L β-glycerosphoric acid, and 10 nmol/L dexamethasone. At the same time, CS-A, CS-B,

CS-C, CS-E, CS-H or CPS was added to the medium. After 16 days passed from when the medium was replaced with the differentiation medium, a culture supernatant was eliminated, the cell was rinsed by PBS (-), dissolved by 1 % Nonidet P-40 (NP-40, registered trademark), and a cell lysate was collected. The cell on a plate was fixed by 15 % formalin, a calcified matrix was dyed by 40 mmol/L alizarin red dyeing (pH 4.2) liquid, and rinsed by pure water. Alizarin red pigments in the plate were dissolved by 10 % formic acid, and light absorption at a wavelength of 415 nm was observed.

The cultured osteoblastic cell MC3T3-E1 became differentiated to an osteoblastic cell by cultivation on a differentiation medium, and formed a calcified matrix which was dyed by alizarin red. CS-E, CS-H and CPS which were polysulfated galactosaminoglycan significantly promoted such formation of a calcified matrix, and the calcified matrix became intensively dyed by alizarin red at 16th day of cultivation. In contrast, it was not observed that CS-A, CS-B or CS-C which was galactosaminoglycan having little ester sulfate groups had such an action (see FIG. 1). It becomes clear that CS-E, CS-H and CPS which are polysulfated galactosaminoglycan enhance formation of a calcified matrix of an osteoblastic cell, thus having a bone/cartilage formation promoting action.

Second Example

For an osteoblastic cell test, CS-A and CS-C used in the first example and chondroitin sulfate D (CS-D, originating from a shark cartilage, D structure content rate was 23.2 %, a sulfur content was 7.1 %, and made by SEIKAGAKU corporation) were purchased and used.

An osteoblastic cell strain MC3T3-E1 was cultured on α-MEM (growth medium) containing 5 % FBS and 10 μg/mL gentamicin. When the cell became confluent, the cell was collected by 0.25 % trypsin solution, and was subjected to seeding to a 96-well plate at $1 \times 10^4$/0.2 mL/well with the growth medium. After 24 hours incubation, the culture medium was replaced with a growth medium (differentiation medium) containing 50 μg/mL ascorbic acid, 10 mmol/L β-glycerophosphoric acid, and 10 nmol/L dexamethasone. At the same time, CS-A, CS-C or CS-D dissolved and diluted by a phosphate buffered saline (PBS (-)) was added to the medium. After 10 days passed from when the medium was replaced with the differentiation medium, a culture supernatant was eliminated, the cell was rinsed by PBS (-), and dissolved by 1 % NP-40 (registered trademark), and a cell lysate was collected. The cell on the plate was fixed by 15 % formalin, a calcified matrix was dyed by 40 mmol/L alizarin red dyeing (pH 4.2) liquid, and then rinsed by pure water. Alizarin red pigments in the plate were dissolved by 10 % formic acid, and light absorption at a wavelength of 415 nm was observed.

The cultured osteoblastic cell MC3T3-E1 became differentiated to an osteoblastic cell by cultivation on the differentiation medium, and formed a calcified matrix which was dyed by alizarin red. CS-D which was polysulfated galactosaminoglycan significantly promoted such formation of a calcified matrix, but it was not observed that CS-A or CS-C which was galactosaminoglycan having little ester sulfate groups had such an action (see FIG. 2). It becomes clear that CS-D which is polysulfated galactosaminoglycan enhances formation of a calcified matrix of an osteoblastic cell, thus having a bone/cartilage formation promoting action.

Third Example

An osteoblastic cell strain MC3T3-E1 was cultured on α-MEM (growth medium) containing 5 % FBS and 10 μg/mL gentamicin. When the cell became confluent, the cell was collected by 0.25 % trypsin solution, and was subjected to seeding to a 48-well plate at $8 \times 10^4$/0.5 mL/well with the growth medium. After 24 hours incubation, the culture medium was replaced with a growth medium (differentiation medium) containing 50 μg/mL ascorbic acid, 10 mmol/L β-glycerophosphoric acid, and 10 nmol/L dexamethasone. At the same time, CS-C or CS-E used in the first example was dissolved and diluted by a phosphate buffered saline (PBS (-)), and then added to the medium. After 8 days passed from when the medium was replaced with the differentiation medium, a culture supernatant was eliminated, the cell was rinsed by PBS (-), and dissolved by 1 % NP-40 (registered trademark), and a cell lysate was collected.

Regarding alkaline phosphatase (ALP) activity, a p-nitrophenol (p-NP) amount created by mixing the cell lysate with p-nitrophenylphosphate (Sigma) and by performing incubation on it for 10 minutes at 37 °C was measured at OD 415 nm, and a p-NP amount was calculated from an analytical curve.

The cultured osteoblastic cell MC3T3-E1 increased an ALP activity which was an osteoblastic cell differentiation marker by cultivation on the differentiation medium.

CS-E which was polysulfated galactosaminoglycan significantly enhanced the ALP activity which was a differentiation marker of an osteoblastic cell, and promoted osteoblastic cell differentiation, but it was not observed that CS-C which was galactosaminoglycan having little ester sulfate groups had such an action (see FIG. 3). It becomes clear that CS-E which is polysulfated galactosaminoglycan enhances an alkaline phosphatase activity of an osteoblastic cell, thus having a bone/cartilage formation promoting action.

Fourth Example

An osteoblastic cell strain MC3T3-E1 was cultured on α-MEM (growth medium) containing 5 % FBS and 10 μg/mL gentamicin. When the cell became confluent, the cell was collected by 0.25 % trypsin solution, and was subjected to seeding to a 96-well plate at $4 \times 10^4$/0.2 mL/well with the growth medium. After 24 hours incubation, the culture medium was replaced with a growth medium (differentiation medium) containing 50 μg/mL ascorbic acid, 10 mmol/L β-glycerophosphoric acid, and 10 nmol/L dexamethasone. At the same time, CS-A, CS-C, CS-D or CS-E used in the first example and the second example was dissolved and diluted by a phosphate buffered saline (PBS (-)), and then added to the medium. After 16 days passed from when the medium was replaced with the differentiation medium, a culture supernatant was eliminated, the cell was rinsed by PBS (-), and dissolved by 1 % NP-40, and a cell lysate was collected.
Regarding a measurement of a DNA amount, PicoGreen dsDNA Quantitation kit (made by Molecular Probes corporation) was used, and a DNA amount was measured from a fluorescence intensity (Ex: wavelength 485 nm, Em: wavelength 535 nm) with a Calf Thymus DNA (Sigma) being as a standard.
Regarding a growth of an osteoblastic cell, CS-E which was polysulfated galactosaminoglycan significantly promoted the cell growth, but it was not observed that CS-A, CS-C and CS-D had such an action (see FIG. 4). It becomes clear that CS-E which is polysulfated galactosaminoglycan enhances growth of an osteoblastic cell, thus having a bone/cartilage formation promoting action.

Fifth Example

Using an MC3T3-E1 cell cultured on calcium phosphate, an action of polysulfated galactosaminoglycan with respect to growth and osteoblastic cell differentiation was checked.
Octacalcium phosphate (OCP) and hydroxyapatite (HA, apaceram, made by pentax corporation) were broken into powders by a mortar, and powders which passed through a stainless mesh (pore size: 53 μm) were used for the test. 1 mg of OCP or HA powders were suspended in 1 mL of distilled water, and added to a 48-well plate 150 μL by 150μ L (0.15 mg/well). It was dried for one night at 80 °C by a dryer, sterilized by 70 % ethanol, and used for the test.
An MC3T3-E1 cell was cultured on α-MEM (growth medium) containing 5 % FBS and 10 μg/mL gentamicin. When the cell became confluent, the cell was collected by 0.25 % trypsin solution, and was subjected to seeding to a 48-well plate at $8 \times 10^4$/0.5 mL/well with the growth medium. After 24 hours incubation, the culture medium was replaced with a growth medium (differentiation medium) containing 50 μg/mL ascorbic acid, 10 mmol/L β-glycerophosphoric acid, and 10 nmol/L dexamethasone, while at the same time CS-E was added. After 12 or 16 days passed from when the medium was replaced with the differentiation medium, a culture supernatant was eliminated, the cell was rinsed by PBS (-), and dissolved by 1 % NP-40 (registered trademark), and a cell lysate was collected. Regarding a measurement of a DNA amount, PicoGreen dsDNA Quantitation kit (made by Molecular Probes corporation) was used, and a DNA amount was measured from a fluorescence intensity (Ex: wavelength 485 nm, Em: wavelength 535 nm) with a Calf Thymus DNA (Sigma) being as a standard.
Regarding an evaluation of calcification, a calcified matrix produced by the cell on the plate was fixed by 15 % formalin, dyed by 40 mmol/L alizarin red dyeing (pH 4.2) liquid, rinsed by deionization water, dissolved by 10 % formic acid, and then light absorption was measured.
Even under an OCP coating or an HA coating condition, CS-E promoted osteoblastic cell differentiation, and promoted production of a calcified matrix which was dyed by alizarin red at 12th day of cultivation (HA) or 16the day thereof (OCP) (see FIGS. 5A and 5B). Moreover, regarding the cell growth, CS-E significantly promoted growth of an osteoblastic cell at 16th day of cultivation under the OCP coating condition (see FIG. 6A). Likewise, under the HA coating condition, it is not significant but a cell growth promoting action was observed (see FIG. 6B). It becomes clear that CS-E has a bone/cartilage formation promoting action when used with a bone filler.

Sixth Example

For a cartilage cell test, chondroitin sulfate E (CS-E, originating from a squid cartilage, E structure was 64.9 %, sulfur content was 8.75 % and made by SEIKAGAKU corporation) was purchased and used. When the test was carried out, CS-E was dissolved and diluted by a phosphate buffered saline (PBS (-)), and then added to a culture medium.
A cartilage cell was separated from a swine knee joint cartilage as follow. A posterior leg of an LWD-kind pig about 1-year-old was purchased from SIMODA animal industry limited private company. After a knee joint was exposed, a joint cartilage of a femur was acquired aseptically. Thereafter, a cell originating from a joint cartilage (hereinafter, "cartilage cell") was separated in accordance with a technique of Mok S. S. et al (J. Biol. Chem., 1994, 269, 33021 to 33027) while modifying some parts of such a technique.
The acquired cartilage was put into DMEM/F-12 culture medium (a culture medium which contained Dulbecco's modified eagle and Ham's F12 at 1:1) containing 0.4 % actinase (made by KAKEN pharmaceutical corporation) and 10

μg/mL gentamicin (made by Invitrogen corporation), and processed for 1 hour at 37 °C while being stirred by a stirrer under a condition where 5 % of $CO_2$ was present. After the culture medium was eliminated, a culture media containing 0.025 % collagenase (made by Roche corporation) and 5 % FBS (made by Biological Industries corporation) was added to a residue, and it was digested for 16 hours at 37 °C under the presence of 5 % of $CO_2$. After digested, the cell was rinsed, collected by 70 μm cell strainer (made by Becton Dickinson corporation), and a number of cells were measured using a counting chamber.

The obtained cell was suspended in a normal saline, containing 2.5 % sodium alginate (alto, made by KAIGEN corporation), at a density of $2 \times 10^6$ number/mL, and three-dimensional culturing was carried out using an alginate gel in accordance with a technique of Flechtenmacher J. et al., (Arthritis Rheum. 1996, 39, 1896 to 1904) while modifying some parts of such a technique.

An alginate sodium solution in which the cell was suspended was charged in a 50 mL disposable syringe (made by TERUMO corporation), and a 22G needle (made by TERUMO corporation) was attached thereto. A piston of the syringe was pressed to drop the solution in 102 mmol/L of $CaCl_2$ (made by Sigma corporation). Through this process, alginate acid turned into a gel in the form of a bead with the cell being contained. A diameter of the alginate acid bead was about 2 mm, and one bead contained about $2 \times 10^4$ numbers of cartilage cells. 10 minutes after the solution was dropped, the bead was rinsed three times by a normal saline, and then cultured on a DMEM/F-12 culture medium, containing 100 ng/ mL of IGF-I, 10 % FBS, 25 μg/mL of L-ascorbic acid, 10 μg/mL of gentamicin, and CS-E of various concentrations, for 6 days at 37 °C using a 24-well culture dish under a condition where 5 % of $CO_2$ was present. The concentrations of CS-E were 4, 20, and 100 μg/mL. The culture media was not replaced. Through the three-dimensional culturing, the cartilage cell produced proteoglycan and type-II collagen which were cartilage matrix components around the cartilage cell.

After the sixth-day three-dimensional culturing, in accordance with the technique of Mok S. S. et al (J. Biol. Chem., 1994, 269, 33021 to 33027), 55 mmol/L of sodium acid citrate buffer solution (pH 6.8) containing 0.15 mol/L of salt and five times as much as the amount of an alginate acid bead was added, stirred, and left for 15 minutes as it was at a room temperature. Alginate acid turned into a gel by Ca ions was dissolved by citric acid. An acquired suspending solution was subjected to centrifuge separation for 5 minutes at 300 × g and at 4 °C, and then a supernatant was disposed. A normal saline was added to a precipitate, stirred, and likewise subjected to centrifuge separation, and then a precipitate which was a cartilage cell with a cartilage matrix was collected.

1 mL of 20 mmol/L HEPES buffer solution (pH 7.5) containing 1 mg/mL of pronase (made by Calbiochem corporation) was added to the acquired cartilage cell, those were airtightly sealed, and the cell was digested for 3 hours at 60 °C. An acquired digestive fluid was used as an analytical sample for a DNA content and a collagen content.

Regarding a DNA content, a fluorescent pigment (Hoechst 33258) made by Hoechst corporation was used, and a DNA content was measured in accordance with a technique of Kim Y-J et al (Anal. Biochem., 1988, 174, 168 to 176) with a Calf Thymus DNA (made by Sigma corporation) being as a standard.

50 μL of the sample acquired through the foregoing process and that of a standard sample with a known concentration were put on 96-well black plate (type 437111, made by Nunc corporation), and the same amount of 100 mM tris-EDTA-NaCl buffer solution (pH 7.4) were added thereto, respectively. 20 mmol/L of HEPES buffer solution (pH 7.5) containing pronase used in the foregoing process was used as a negative contrast sample. Subsequently, 100 μL of 5 μg/mL Hoechst 33258 solution dissolved in the same buffer solution was added to each sample, and those were stirred for 1 minute by a shaking device. Thereafter, using a fluorescent plate reader (type Twinkle LB970, made by Berthold corporation), measurement were carried out at an excitation wavelength, 360 nm, a fluorescence wavelength, and 460 nm. A standard curve was made on the basis of a fluorescent intensity of the standard sample, and a DNA content in each sample was calculated from the standard curve.

Regarding a collagen content, a collagen content was calculated by measuring hydroxyproline in a hydrolyzed sample in accordance with a technique of Woessner JF. Jr. (Arch. Biochem. Biophys., 1961, 93, 440 to 447).

100 μL of the sample acquired through the foregoing process was put on a glass vial container (type 08-CPV, made by Chromacol corporation), the same amount of hydrochloric acid was added, airtightly sealed by an aluminum cap with a tetrafluoroethylene-resin-made septum (type 8-AC-TST1, made by Chromacol corporation), and subjected to hydrolysis for 16 hours at 120 °C. 20 mmol/L of HEPES buffer solution (pH 7.5) containing pronase used in the foregoing process was used as a negative contrast sample. Moreover, a swine type-II collagen solution (made by Chondrex corporation) having a known content was also used as a standard sample.

100 μL of the sample having undergone hydrolysis was put on a 0.8 mL deep well plate (type AB-0765, made by ABgene House corporation) having a content, and then subjected to pressure reduction and drying in a vacuum desiccator in which sodium hydroxide was present together. Distilled water was added to the dried sample, and light absorption at 557 nm was measured after the sample was colored in accordance with a technique of Woessner JF. Jr.. A standard curve was created on the basis of the light absorption of the standard sample, and a collagen content in each sample was calculated from the standard curve.

FIG. 7 shows a collagen content per twenty alginate gel beads, and FIG. 8 shows a collagen content per DNA.

100 μg/mL of CS-E significantly increased a collagen content per twenty alginate gel beads and a collagen content per DNA. That is, it is suggested that CS-E has an action of promoting collagen synthesis, i.e., a cartilage formation promoting action to a cartilage cell.

Table 1 shows a disaccharide analysis result of sulfated galactosaminoglycan used in the present invention. The analysis was carried in accordance with a technique of Yoshida K., et al (Anal Biochem., 177, 327 to 332, 1989). Since there is no enzyme which can be decomposed into a disaccharide, CPS could not be analyzed. According to this technique, even if constituent uronic acid of sulfated galactosaminoglycan is iduronic acid, the same unsaturated disaccharide as that of the case of glucuronic acid can be provided. For example, a structure (dermatan sulfate structure) having iduronic acid as constituent uronic acid and having an ester sulfate group at the position 4 of N-acetylgalactosamine provides ΔDi-4S, and a structure (chondroitin sulfate H structure) having iduronic acid as constituent uronic acid and having an ester sulfate group at the position 4 and the position 6 of N-acetylgalactosamine provides ΔDi-$S_E$.

[Table 1]

| Sulfated Galactosaminoglycan | Disaccharide Structure (%) | | | | | | |
|---|---|---|---|---|---|---|---|
| | ΔDi-0S | ΔDi-6S | ΔDi-4S | ΔDi-$S_D$ | ΔDi-$S_B$ | ΔDi-$S_E$ | ΔDi-TriS |
| CS-A | 1.4 | 18.0 | 78.4 | 2.0 | 0.0 | 0.2 | 0.0 |
| CS-B | 0.5 | 1.0 | 91.7 | 0.3 | 6.5 | 0.0 | 0.0 |
| CS-C | 0.6 | 89.1 | 6.3 | 3.5 | 0.0 | 0.5 | 0.0 |
| CS-D | 0.7 | 70.1 | 2.8 | 23.2 | 0.0 | 3.2 | 0.0 |
| CS-E | 8.2 | 10.2 | 16.7 | 0.0 | 0.0 | 64.9 | 0.0 |
| CS-H | 5.5 | 6.1 | 15.0 | 1.8 | 7.9 | 42.1 | 21.6 |

Abbreviations of individual unsaturated disaccharides mean the following structures.
ΔDi-OS: 2-acetamide-2-deoxy-3-O-(β-D-gluco-4-enopyranosyl uronic acid)-D-galactose
ΔDi-6S: 2-acetamide-2-deoxy-3-O-(β-D-gluco-4-enopyranosyl uronic acid)-6-O-sulfo-D-galactose
ΔDi-4S: 2-acetamide-2-deoxy-3-O-(β-D-gluco-4-enopyranosyl uronic acid)-4-O-sulfo-D-galactose
ΔDi-$S_D$: 2-acetamide-2-deoxy-3-O-(2-O-sulfo-β-D-gluco-4-enopyranosyl uronic acid)-6-O-sulfo-D-galactose
ΔDi-$S_B$: 2-acetamide-2-deoxy-3-O-(2-O-sulfo-β-D-gluco-4-enopyranosyl uronic acid)-4-O-sulfo-D-galactose
ΔDi-$S_E$: 2-acetamide-2-deoxy-3-O-(β-D-gluco-4-enopyranosyl uronic acid)-4,6-di-O-sulfo-D-galactose
ΔDi-TriS: 2-acetamide-2-deoxy-3-O-(2-O-sulfo-(3-D-gluco-4-enopyranosyl uronic acid)-4,6-di-O-sulfo-D-galactose

Industrial Applicability

The present invention relates to a bone/cartilage formation promoting agent, and can be used in the field of medicinal drugs or the like.

## Claims

1. A bone/cartilage formation promoting agent comprising, as an active ingredient, sulfated galactosaminoglycan or a pharmacologically acceptable salt thereof, the sulfated galactosaminoglycan containing greater than or equal to 0.6 numbers of ester sulfate groups on average per constituent monosaccharide.

2. The bone/cartilage formation promoting agent according to claim 1, wherein sulfated galactosaminoglycan is sulfated galactosaminoglycan containing an N-acetylchondrosine or N-acetyldermosine structure.

3. The bone/cartilage formation promoting agent according to claim 1 or 2, wherein sulfated galactosaminoglycan has a structure of any one of formulas 1 to 4.

[Chemical Formula 1]

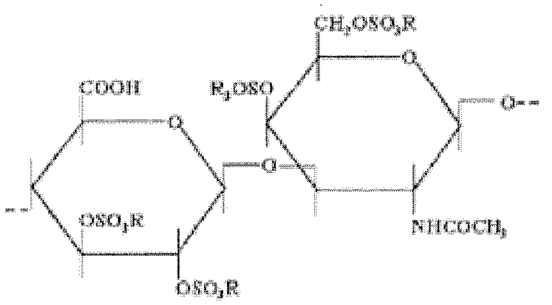

( Formula 1 )

[Chemical Formula 2]

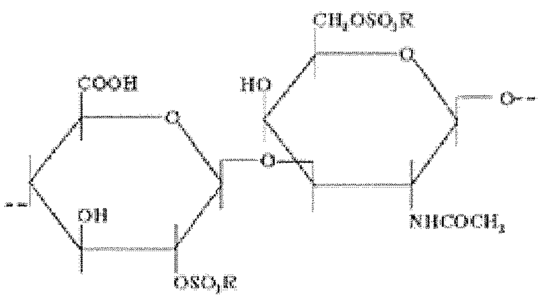

( Formula 2 )

[Chemical Formula 3]

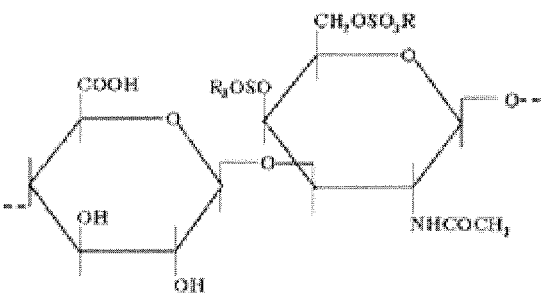

( Formula 3 )

[Chemical Formula 4]

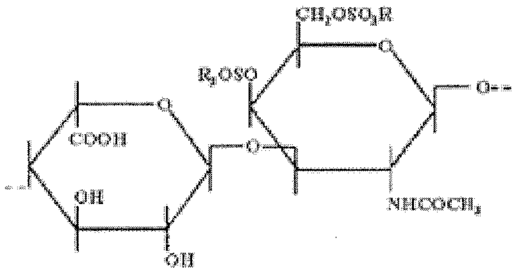

( Formula 4 )

(where C, O, H, N and S in formulas represent a carbon atom, an oxygen atom, a hydrogen atom, a nitrogen atom, and a sulfur atom, respectively, and R represents a hydrogen atom or a substituent thereof.)

**4.** The bone/cartilage formation promoting agent according to any one of claims 1 to 3, wherein a molecular weight of sulfated galactosaminoglycan is greater than or equal to 1000 Da.

**5.** The bone/cartilage formation promoting agent according to any one of claims 1 to 4, wherein a content rate of the structure of any one of formulas 1 to 4 is greater than or equal to 20 mol%.

[Chemical Formula 5]

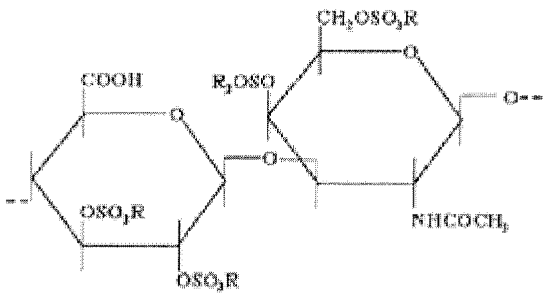

( Formula 1 )

[Chemical Formula 6]

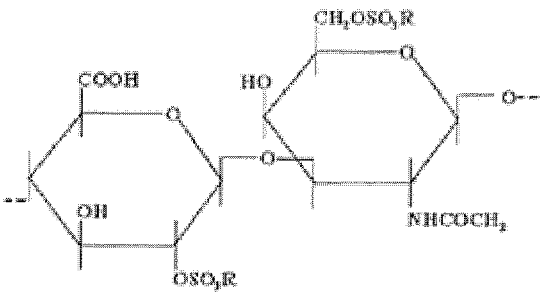

( Formula 2 )

[Chemical Formula 7]

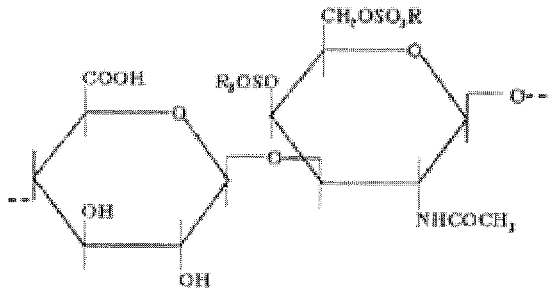

( Formula 3 )

[Chemical Formula 8]

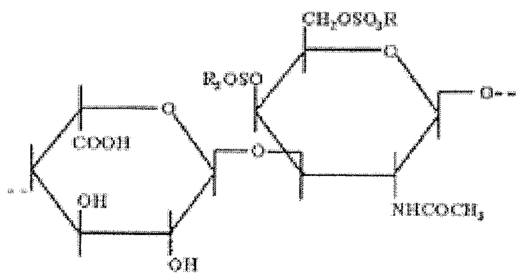

( Formula 4 )

(where C, O, H, N and S in formulas represent a carbon atom, an oxygen atom, a hydrogen atom, a nitrogen atom, and a sulfur atom, respectively, and R represents a hydrogen atom or a substituent thereof.)

**6.** A bone/cartilage formation promoting agent comprising, as an active ingredient, sulfated galactosaminoglycan originating from a cartilage of cephalopoda or selachii, or a chorda dorsalis of agnathonae.

**7.** The bone/cartilage formation promoting agent according to any one of claims 1 to 6, further comprising, as an additive, a factor of promoting bone formation or a bone filler.

**8.** The bone/cartilage formation promoting agent according to claim 7, wherein the factor of promoting bone formation or the bone filler is at least one substance selected from BMP, TGF-β, FGF, IGF, insulin, PDGF, HGF, midkine, pleiotrophin, collagen, gelatin, proteoglycan, fibronectin, osteocalcin, osteopontin, osteonectin, bone sialoprotein, hydroxyapatite, dicalcium phosphate anhydride, dicalcium phosphate dehydrate, α-tricalcium phosphate, amorphous calcium phosphate, octacalcium phosphate, β-tricalcium phosphate, PLLA, PLGA, titanium, decalcified bone, and autogeneous bone.

**9.** A pharmaceutical composition comprising the bone/cartilage formation promoting agent of any one of claims 1 to 8 for cartilage formation failure and cartilage formation.

**10.** A method of using sulfated galactosaminoglycan or a pharmacologically acceptable salt thereof for promoting bone/cartilage formation, the sulfated galactosaminoglycan containing greater than or equal to 0.6 numbers of ester sulfate groups on average per constituent monosaccharide.

**11.** A method of using sulfated galactosaminoglycan or a pharmacologically acceptable salt thereof for promoting cartilage formation, the sulfated galactosaminoglycan containing greater than or equal to 0.6 numbers of ester sulfate groups on average per constituent monosaccharide.

[FIG. 1]

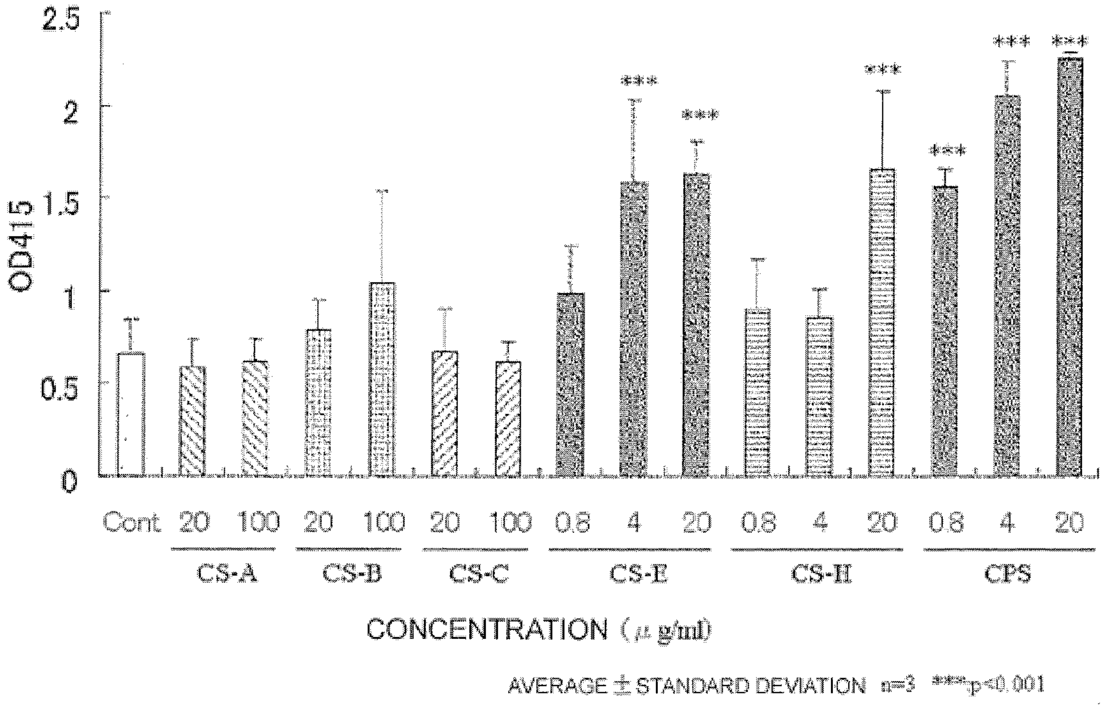
2.5
2
1.5
1
0.5
0
OD415
***
***
***
***
***
***
Cont
20
100
20
100
20
100
0.8
4
20
0.8
4
20
0.8
4
20
CS-A
CS-B
CS-C
CS-E
CS-H
CPS
CONCENTRATION (μg/ml)
AVERAGE ± STANDARD DEVIATION n=3 ***:p<0.001

[FIG. 2]

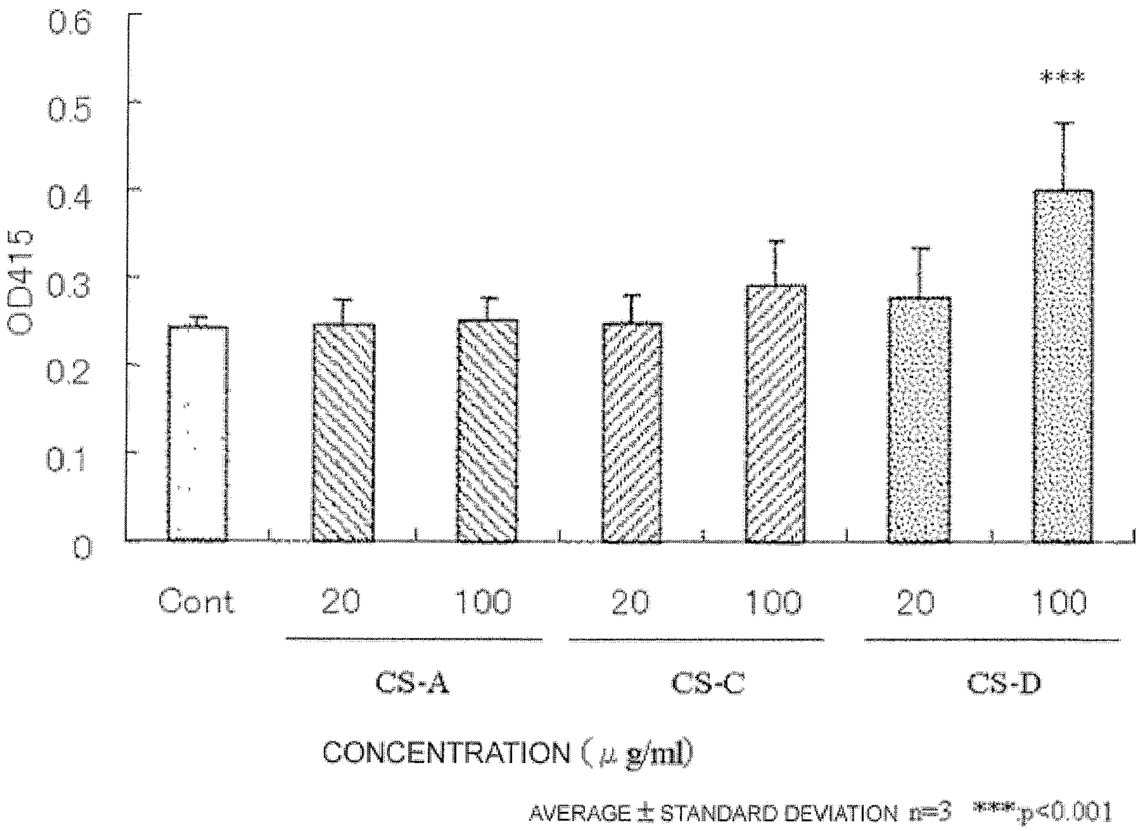
0.6
0.5
0.4
0.3
0.2
0.1
0
OD415
***
Cont
20
100
20
100
20
100
CS-A
CS-C
CS-D
CONCENTRATION (μ g/ml)
AVERAGE ± STANDARD DEVIATION n=3 ***:p<0.001

[FIG. 3]

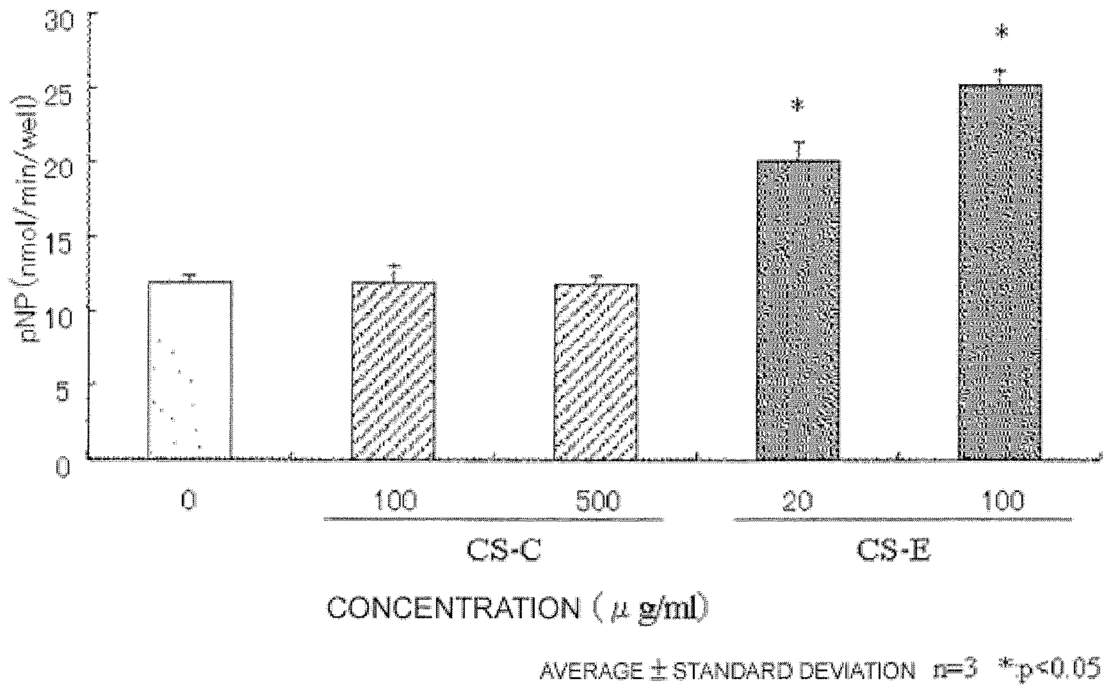
pNP (nmol/min/well)
30
25
20
15
10
5
0
*
*
0
100
500
20
100
CS-C
CS-E
CONCENTRATION (μ g/ml)
AVERAGE ± STANDARD DEVIATION n=3 *:p<0.05

[FIG. 4]

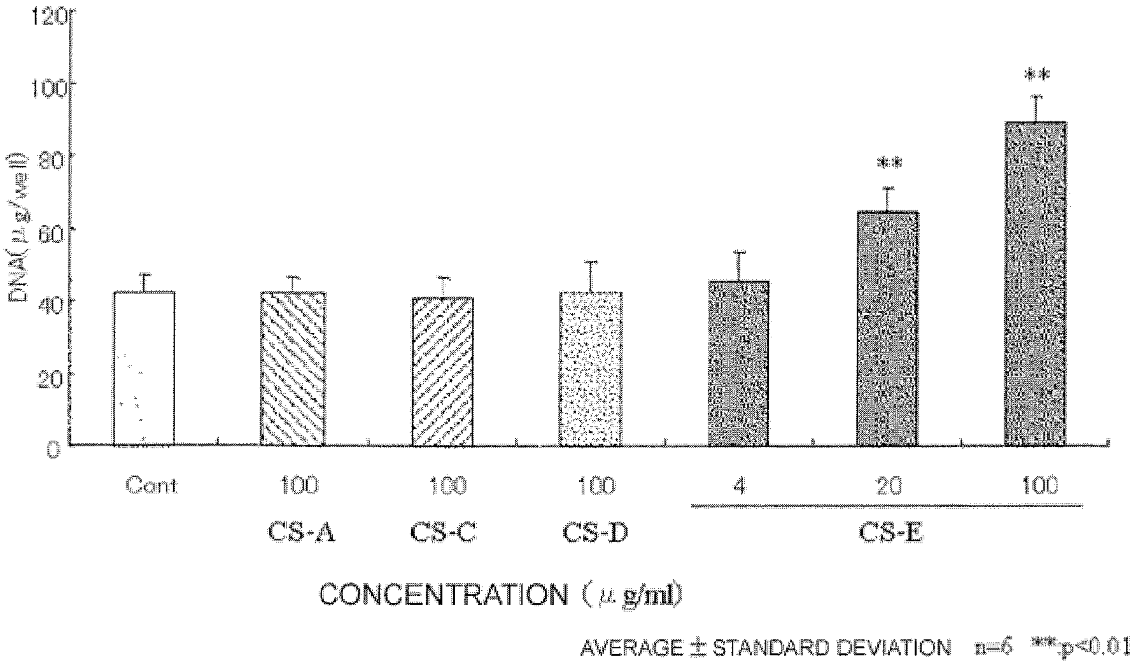
120
100
80
60
40
20
0
DNA(μg/well)
**
**
Cont
100
100
100
4
20
100
CS-A
CS-C
CS-D
CS-E
CONCENTRATION (μg/ml)
AVERAGE ± STANDARD DEVIATION n=6 **:p<0.01

[FIG. 5]

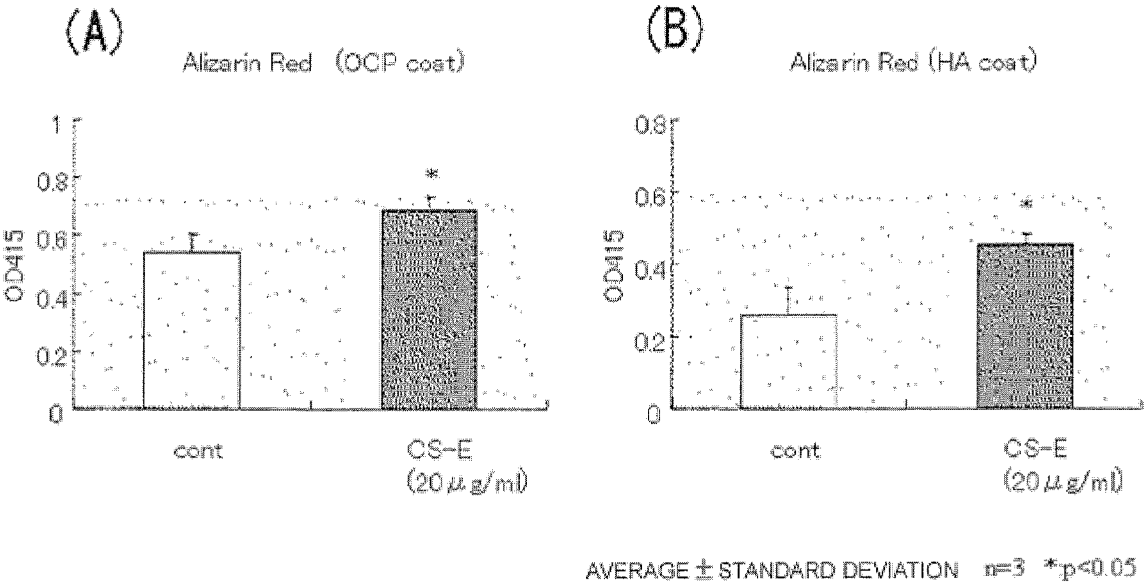
(A)
Alizarin Red (OCP coat)
OD415
1
0.8
0.6
0.4
0.2
0
*
cont
CS-E
(20 μ g/ml)
(B)
Alizarin Red (HA coat)
OD415
0.8
0.6
0.4
0.2
0
*
cont
CS-E
(20 μ g/ml)
AVERAGE ± STANDARD DEVIATION n=3 *:p<0.05

[FIG. 6]

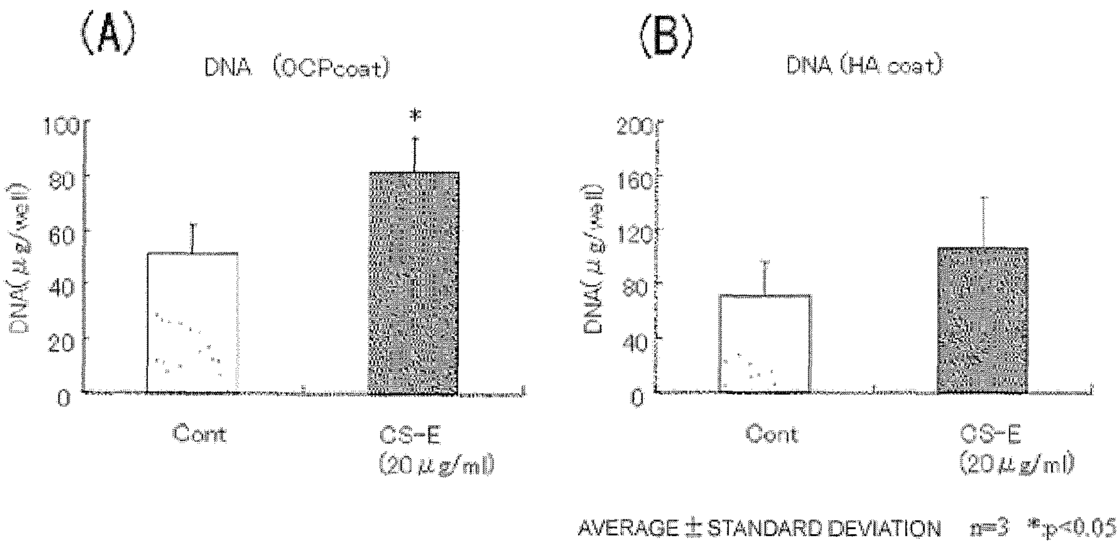
(A)
DNA (OCPcoat)
DNA(μg/well)
100
80
60
40
20
0
*
Cont
CS-E
(20μg/ml)
(B)
DNA (HA coat)
DNA(μg/well)
200
160
120
80
40
0
Cont
CS-E
(20μg/ml)
AVERAGE ± STANDARD DEVIATION n=3 *:p<0.05

[FIG. 7]

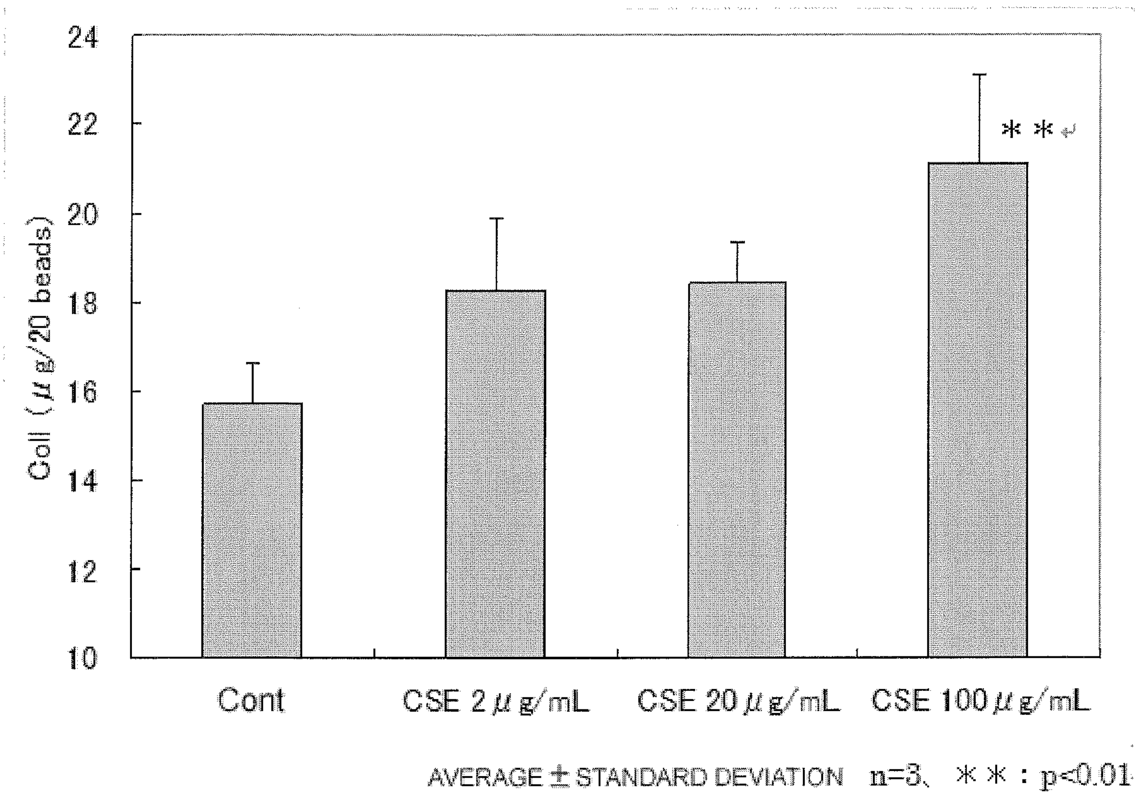
24
22
20
18
16
14
12
10
Coll (μg/20 beads)
＊＊
Cont
CSE 2 μg/mL
CSE 20 μg/mL
CSE 100 μg/mL
AVERAGE ± STANDARD DEVIATION n=3、＊＊: p<0.01

[FIG. 8]

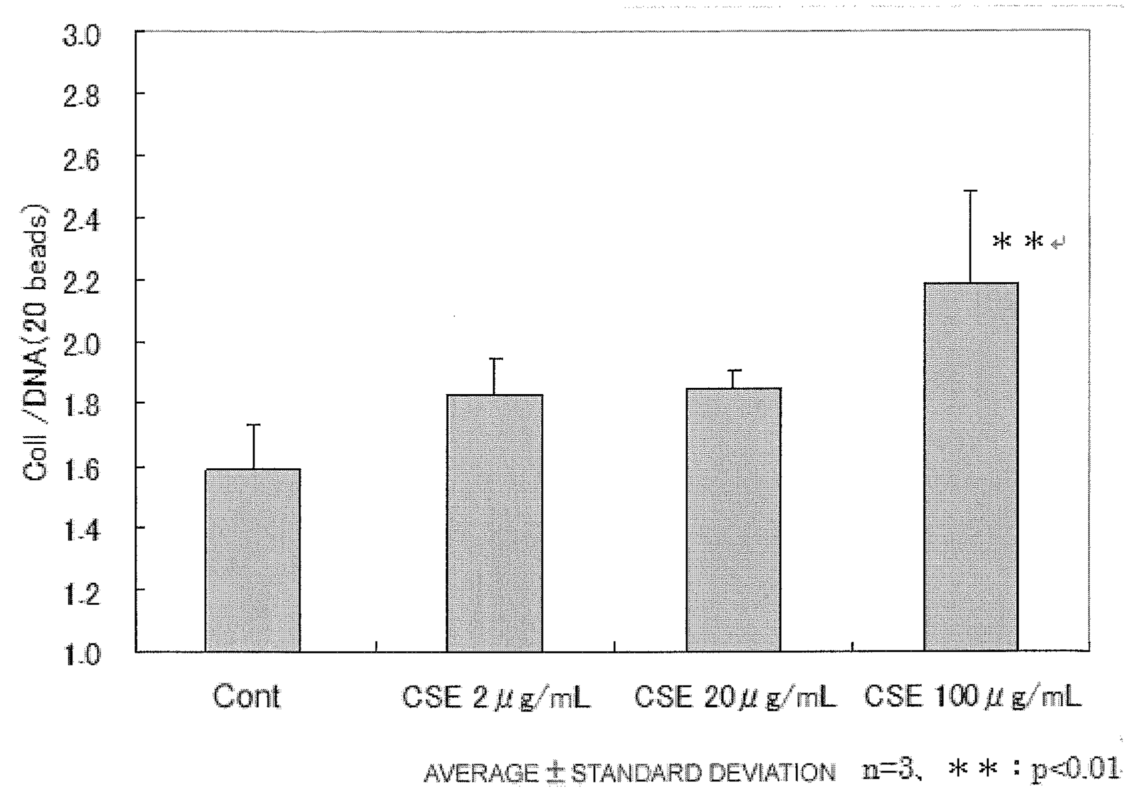
3.0
2.8
2.6
2.4
2.2
2.0
1.8
1.6
1.4
1.2
1.0
Coll /DNA(20 beads)
＊＊
Cont
CSE 2 μg/mL
CSE 20 μg/mL
CSE 100 μg/mL
AVERAGE ± STANDARD DEVIATION n=3、＊＊：p<0.01

**INTERNATIONAL SEARCH REPORT**

International application No.
PCT/JP2008/000316

A. CLASSIFICATION OF SUBJECT MATTER
*C08B37/00*(2006.01)i, *A61K31/726*(2006.01)i, *A61P19/10*(2006.01)i

According to International Patent Classification (IPC) or to both national classification and IPC

B. FIELDS SEARCHED

Minimum documentation searched (classification system followed by classification symbols)
C08B37/00, A61K31/726, A61P19/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 2004-501682 A (Biosyntech Canada Inc.), 22 January, 2004 (22.01.04), Claims 1, 4 to 8, 27 & WO 2002/000272 A2 & US 2002/0082220 A1 & EP 1294414 A | 1-9 |
| Y | SUGAHARA K, YAMADA S, Structure and Function of Oversulfated Chondroitin Sulfate Variants. Unique Sulfation Patterns and Neuroregulatory Activities., Trends Glycoscience Glycotechnology, 2000, Vol.12, No.67, Page.321-349 | 1-9 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 March, 2008 (25.03.08) | 08 April, 2008 (08.04.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.
PCT/JP2008/000316

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2000-514698 A (Orquest, Inc.), 07 November, 2000 (07.11.00), Claims 1, 4 to 5, 16, 18 to 19 & US 5866165 A & EP 994694 A & WO 1998/031345 A1 | 1-9 |
| Y | JP 08-229114 A (Mitsubishi Materials Corp.), 10 September, 1996 (10.09.96), Claim 1 (Family: none) | 1-9 |
| Y | JP 08-229113 A (Mitsubishi Materials Corp.), 10 September, 1996 (10.09.96), Claim 1 (Family: none) | 1-9 |
| Y | JP 05-070113 A (JGC Corp.), 23 March, 1993 (23.03.93), Claim 1 (Family: none) | 1-9 |
| Y | JP 2006-167445 A (Nippon Meat Packers, Inc.), 29 June, 2006 (29.06.06), Claim 1 (Family: none) | 1-9 |
| P,A | JP 2007-161688 A (Hiroshima University), 28 June, 2007 (28.06.07), Claims 1, 3 (Family: none) | 1-9 |

Form PCT/ISA/210 (continuation of second sheet) (April 2007)

**INTERNATIONAL SEARCH REPORT**

International application No.
PCT/JP2008/000316

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 10-11
because they relate to subject matter not required to be searched by this Authority, namely:
The inventions as set forth in claims 10 to 11 pertain to methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H0570113 A
- JP 2006167445 A
- JP H08229114 A
- JP H08229113 A


### Non-patent literature cited in the description


- **Bouvier M. et al.** *Arch Oral Biol,* 1990, vol. 35 (4), 301-309
- **Hausser H-J et al.** *Cell Biochem,* 2004, vol. 91, 1062-1073
- **Zhao B. et al.** *J Biol Chem,* 2006, vol. 281 (32), 23246-53
- **Bitter T. et al.** *Anal Biochem,* 1962, vol. 4, 330-334
- **Yoshida K et al.** *Anal Biochem,* 1989, vol. 177, 327-332
- **Wozney JM. et al.** *Prog Growth Factor Res,* 1989, vol. 1 (4), 267-280
- **Joyce ME. et al.** *J Cell Biol,* 1990, vol. 110 (6), 2195-2207
- **Mayahara H. et al.** *Growth Factors,* 1993, vol. 9 (1), 73-80
- **Mueller K. et al.** *Am J Physiol,* 1994, vol. 267, E1-6
- **Cornish J. et al.** *Calcif Tissue Int,* 1996, vol. 59 (6), 492-495
- **Vikjaer D. et al.** *Eur J Oral Sci,* 1997, vol. 105 (1), 59-66
- **Amano O.** *Arch Oral Biol,* 1999, vol. 44 (11), 935-946
- **Ohta S. et al.** *J Bone Miner Res,* 1999, vol. 14 (7), 1132-1144
- **Imai S. et al.** *J Cell Biol,* 1998, vol. 143 (4), 1113-1128
- **Saadeh PB. et al.** *J Craniofac Surg,* 2001, vol. 12 (6), 573-579
- **Gomes RR. Jr et al.** *Connect Tissue Res,* 2003, vol. 44 (1), 196-201
- **Mok S. S. et al.** *J. Biol. Chem.,* 1994, vol. 269, 33021-33027
- **Flechtenmacher J. et al.** *Arthritis Rheum.,* 1996, vol. 39, 1896-1904
- **Kim Y-J et al.** *Anal. Biochem.,* 1988, vol. 174, 168-176
- **Woessner JF. Jr.** *Arch. Biochem. Biophys.,* 1991, vol. 93, 440-447
- **Yoshida K. et al.** *Anal Biochem.,* 1989, vol. 177, 327-332